(19) Europäisches Patentamt

European Patent Office

Office européen des brevets

(11) **EP 0 535 627 B1**

(12) **EUROPEAN PATENT SPECIFICATION**

(45) Date of publication and mention
of the grant of the patent:
**02.06.1999 Bulletin 1999/22**

(51) Int Cl.⁶: **C08B 30/18**, C12P 19/14,
A23L 1/308, A23L 1/09,
A23L 2/42

(21) Application number: **92116722.7**

(22) Date of filing: **30.09.1992**

(54) **Alcoholic beverage containing an indigestible dextrin**

Alkoholisches Getränk enthaltend ein unverdauliches Dextrin

Boisson alcoolique contenant une dextrine indigestible

(84) Designated Contracting States:
**AT BE CH DE DK ES FR GR IT LI LU NL SE**

(30) Priority: **30.09.1991 JP 280499/91**
**25.11.1991 JP 336314/91**

(43) Date of publication of application:
**07.04.1993 Bulletin 1993/14**

(73) Proprietor: **MATSUTANI CHEMICAL INDUSTRIES CO. LTD.**
**Itami-shi, Hyogo-ken (JP)**

(72) Inventors:
• **Ohkuma, Kazuhiro**
**Sanda-shi, Hyogo-ken (JP)**
• **Hanno, Yoshio**
**Itami-shi, Hyogo-ken (JP)**
• **Inada, Kazuyuki**
**Takarazuka-shi, Hyogo-ken (JP)**
• **Matsuda, Isao**
**Itami-shi, Hyogo-ken (JP)**
• **Katta, Yasuo**
**Kawanishi-shi, Hyogo-ken (JP)**

(74) Representative: **Gillard, Marie-Louise et al**
**Cabinet Beau de Loménie**
**158, rue de l'Université**
**75340 Paris Cédex 07 (FR)**

(56) References cited:
**EP-A- 0 368 451          EP-A- 0 444 891**

**Description**

BACKGROUND CF THE INVENTION

1. Field of the invention

The present invention relates to an alcoholic beverage containing indigestible dextrins which are prepared by heat-treating corn starch with addition of an acid and hydrolyzing the resulting starch with alpha-amylase and glucoamylase and which contain dietary fiber and have a low caloric value.

2. Description of the Prior Art

Pyrodextrins are prepared by heating a starch containing several percent of water in the presence or absence of acid. These dextrins include British gum which is obtained by heating the starch at 135 to 218°C in the absence of acid for 10 to 20 hours, white dextrin which is prepared by heating the starch at 79 to 121°C in the presence of acid for 3 to 8 hours, and yellow dextrin which is prepared similarly by heating the starch at 150 to 220°C with addition of acid for 6 to 18 hours.

It is known that these dextrins are consisted of glucose, the component of starch, which consists primarily of $1 \rightarrow 4$ and $1 \rightarrow 6$ glycosidic linkages and contains very small amounts of $1 \rightarrow 3$ and $1 \rightarrow 2$ glycosidic linkages.

The proportions of these glycosidic linkages are disclosed only in J.D. Geerdes et al., J. Am. Chem. Soc., Vol. 79, 4209(1957), G.M. Christensen et al., J. Am. Chem. Soc., Vol. 79, 4492(1957) and the literature mentioned below. Methylation analysis of pyrodextrin obtained by heat-treating commercial corn starch with addition of hydrochloric acid reveals that the pyrodextrin comprises at least 57.3% of $1 \rightarrow 4$ glycosidic linkage fraction (2,3,6-Tri-O-Methyl-D-glucose), 2.6% of $1 \rightarrow 6$ glycosidic linkage fraction (2,3,4-Tri-O-Methyl-D-glucose), up to 1.2% of $1 \rightarrow 3$ glycosidic linkage fraction (2,4,6-Tri-O-Methyl-D-glucose), 6.3% of a fraction having both $1 \rightarrow 4$ and $1 \rightarrow 6$ linkages (2,3-Di-O-Methyl-D-glucose) and about 20% of a fraction having other glycosidic linkages.

Further R.L. Whistler and E.F. Paschall, Starch Chemistry & Technology, Vol. 1, 430(1965) makes reference to analyzed values of linkage types constituting heat-treated amylopectin and heat-treated amylose which were obtained by separating corn starch into amylopectin and amylose fractions and individually heating the fractions with addition of an acid. The analyzed values were obtained for the heat-treated fractions which were prepared by gelatinizing the starch, then separating the starch into the two fractions and heating the fractions. The form of powder heat-treated therefore differs from that of natural starch, so that the values can not be used directly for comparison. However, in view of the fact that the ratio between the two fractions of usual corn starch is about 8:2, the analyzed values, when calculated for corn starch, correspond to 67% of $1 \rightarrow 4$ glycosidic linkage fraction (2,3,6-Tri-O-Methyl-D-glucose), 2.7% of $1 \rightarrow 3$ glycosidic linkage fraction (2,4,6-Tri-O-Methyl-D-glucose) and 7.8% of a fraction having both $1 \rightarrow 4$ and $1 \rightarrow 6$ glycosidic linkages (2,3 Di-O-Methyl-D-glucose).

Tomasik, P. and Wiejak, S., Advance in Carbohydrate Chemistry, Vol. 47, 279-343(1990) generally describes the latest information as to processes for preparing pyrodextrins.

When analyzed, however, any of commercial pyrodextrins was found to be up to 30% in indigestible content, up to 3% in dietary fiber content, at least 3.1 kcal/g in caloric value 1 and at least 3.1 kcal/g in caloric value 2. When starch was heated under altered conditions to increase these contents, it was possible to increase the indigestible content to about 60% and the dietary fiber content to about 30% and to decrease the caloric value 1 to about 2.7 kcal/g and the caloric value 2 to about 2 kcal/g, whereas the product then contained an increased amount of colored substance, had a stimulative odor, therefore required refining and was not practically useful because of extreme difficulties encountered in refining the product. Accordingly, it is impossible to obtain a dextrin which is at least 75% in indigestible content, at least 20% in dietary fiber content, up to 2.6 kcal/g in caloric value 1 and up to 2 kcal/g in caloric value 2 as contemplated by the present invention.

With respect to the enzymic hydrolysis of pyrodextrins, B. Brimhall, Ind. Eng. Chem., 36, 72(1944) discloses that when so-called British gum prepared by heating starch in the absence of acid is hydrolyzed with alpha-amylase, the limit of decomposition is 3.5% calculated as maltose, i.e., about 7.4 calculated as DE.

Further U.S. Patent No. 3,974,032 discloses a hydrolyzate which is prepared from a pyrodextrin obtained with addition of hydrochloric acid and having a degree of branching of 7 to 16% by hydrolyzing the pyrodextrin at 60 to 85°C to DE of 9 to 20 with alpha-amylase and which is up to 20 in the ratio of weight average molecular weight to number average molecular weight and contains up to 20% of oligosaccharides having a degree of polymerization of 200. However, the patent discloses nothing about hydrolysis with glucoamylase or about dietary fiber.

With improvements in living standards in Japan in recent years, eating habits have changed and become similar to those of American and European people. This trend has resulted in a lengthened average life span and a rapidly aging society with marked increases in degenerative diseases. Manifestly, people have become health-oriented.

Attention has, therefore, been directed to dietary fibers and oligosaccharides enhance the function of foods and livestock feeds in that these materials are known to alleviate constipation and other desired biological regulatory functions.

Indigestible substances, such as dietary fibers and oligosaccharides, exhibit various modes of behavior in the digestive tracts producing physiological effects on the living body. First in the upper digestive tract, water-soluble dietary fibers slow the transport of food and delay the absorption of nutrients. Delayed absorption of sugar, for example, suppresses the rise in blood sugar value, consequently lowering insulin requirements. Further, excretion of bile acid is promoted, diminishing the sterol group in the body thereby lowering the cholesterol level of the serum. Other physiological effects through the endocrine system of the body are also reported.

Another feature of these indigestible substances is they are not digested or absorbed by the digestive tract, including the small intestine and reach the large intestine. On reaching the large intestine, oligosaccharides and dietary fibers are partly acted on by enterobacteria yielding short-chain fatty acids, intestinal gases, vitamins, etc. Acidification of the intestinal environment by the short-chain fatty acids condition the intestine. It has also been reported that when absorbed, these short-chain fatty acids are metabolized to provide energy and, simultaneously, inhibit the synthesis of cholesterol. Therefore, ingestible substances are necessary in obtaining these desired physiological effects.

A "dietary fiber hypothesis" suggested by Trowell and Burkitt epidemiologically revealed that there is a negative correlation between the intake of dietary fibers and the onset of non-infectious diseases such as cholelithiasis, ischemic heart diseases, cancer of the large intestine, etc. Thus, insufficient ingestion of dietary fibers is thought to be a cause of degenerative diseases which are said to be diseases of the Western type. The dietary fibers are defined as the "whole group of indigestible components of foods which are not digestible by human digestive enzymes" and are classified into insoluble dietary fibers and water-soluble dietary fibers according to the solubility in water. Of these, water-soluble dietary fibers have attracted attention as materials for functional foods and livestock feeds because of their great physiological function.

For example, it is said that high viscosities inhibit diffusion of sugar, resulting in delayed absorption of sugar and reduction in the rise of blood sugar value, consequently lowering insulin necessity. Further it is said that promoted excretion of bile acid into feces by water-soluble dietary fibers diminishes cholesterol in the serum, and that after reaching the large intestine, the dietary fibers are acted on by enterobacteria to produce lactic acid and acetic acid with these organic acids lowering the pH within the large intestine and preventing cancer of the large intestine.

Examples of such water-soluble dietary fibers include guar gum, glucomannan, pectin and like natural gums which have high viscosity which are difficult to ingest singly in a large amount. Further the addition of these fibers to processed foods encounters problems in preparing the food and presents difficulties with respect to texture. It has therefore long been desired to provide dietary fibers of low viscosity which have the same physiological functions as the above fibers, are easy to ingest and are user-friendly in preparing processed foods.

In recent years in Japan, processed foods, precooked foods, fast foods and the like have found wider use with the maturity of economical environments and the resulting improvements in food processing techniques and distribution techniques. With this trend, diversified information as to the ingestion of foods has become available, and eating habits to fulfill the nutrient requirements are changing to health-oriented dietary habits contemplated for the prevention of nutrition disorders and degenerative diseases due to eating habits. Especially, people of middle or advanced age and young women have much need for low caloric foods, so that low caloric sweeteners and bulking agents for strong sweetening agents have been developed. Among these, low caloric sweeteners include various indigestible oligosaccharides and sugar alcohols, which nevertheless have many problems with respect to the quality, degree of sweetness, oligosaccharide content and likelihood of causing laxation.

The bulking agent available for use with strong sweetening agents such as aspartame is polydextrose only, whereas polydextrose is ingestible in a limited amount, tastes bitter in an acid condition, is hygroscopic and therefore has problems. In view of the situation described, it has been desired to provide a low caloric bulking agent which fulfills the requirements for use as a food and which is usable for sweeteners and the like with safety.

On the other hand, starch is used in large quantities in various processed foods as a food material. Useful food materials of these types include starch and starch products such as pregelatinized starch, pyrodextrin and its derivatives, glucose, corn syrup solids and maltodextrin. However, a majority of these starch products are not higher than 5% in the content of indigestible component and at least 3.9 kcal/$g$ in caloric value, so that among starches and like materials, only pyrodextrin appears useful as a dietary fiber and low caloric material. Heat-treated starch (pyrodextrin) will hereinafter be referred to merely as "dextrin".

SUMMARY OF THE INVENTION

Accordingly, the main object of the present invention is to provide an alcoholic beverage containing an indigestible dextrin which is diminished in the amount of colored substance and stimulative odor and which contains at least 75% of an indigestible component and at least 13% of dietary fiber and is up to 2.6 kcal/g in caloric value 1 and up to 2 kcal/g in caloric value 2. Preferably, the portion of the dextrin other than glucose contains at least 90% of an indigestible component and at least 20% of dietary fiber and is up to 1.8 kcal/g in caloric value 1 and up to 1.2 kcal/g in caloric value 2.

The term "dextrin" used merely as such hereinafter means a heat-treated starch (pyrodextrin).

We conducted research on processes for preparing dextrins, hydrolysis processes and processes for preparing indigestible dextrins from dextrins. Based on the results obtained, we filed a patent application for an invention entitled "Process for Preparing Indigestible Dextrin." Our research subsequently carried out on the physiological activities of this dextrin revealed that the dextrin had an intestine conditioning effect, effect to ameliorating hypercholesterolemia, effect to lower insulin requirements, hypotensive effect and low caloric value, i.e., effects similar to those of dietary fibers. Based on the finding, we filed a patent application for the dextrin as a food composition.

We have also investigated the correlation of the structure of the dextrin with the content of indigestible component or dietary fiber and with the caloric value thereof, and found that the contents of indigestible component and dietary fiber of the dextrin are in inverse proportion to the amount of $1 \rightarrow 4$ glycosidic linkages in the dextrin among other glycosidic linkages therein, and that the caloric value is in proportion to the amount of $1 \rightarrow 4$ glycosidic linkages in pyrodextrin among other glycosidic linkages therein. We have further conducted detailed research.

The research thus conducted on various pyrodextrins indicates that the contents of indigestible component and dietary fiber and the caloric value are closely related with the amounts of glycosidic linkages such as $1 \rightarrow 4$ glycosidic linkages and with the average molecular weight of the dextrin, consequently affording equations representing high degrees of correlation by statistical numerical analysis. However, the commercial pyrodextrins obtained by the prior art are as low as 5 to 30% in the content of indigestible component and 3 to 12% in dietary fiber content and as high as 3.3 to 3.9 kcal/g in caloric value 1 and 3.1 to 3.85 kcal/g in caloric value 2. Although attempts are made to produce an improved pyrodextrin by a longer period of reaction at a high temperature, the product contains a colored substance, releases a stimulative odor and is in no way practically useful.

We have further conducted research to give increased contents of indigestible component and dietary fiber and found the following.

1) When pyrodextrin is hydrolyzed with alpha-amylase and glucoamylase, the resulting glucose and monosaccharides (which consist primarily of glucose and will therefore be referred to as "glucose" hereinafter) can be substantially separated off by ion exchange resin chromatography.
2) When at least one-half of digestible glucose is removed from the hydrolyzate to obtain an indigestible fraction, the fraction contains at least 75% of an indigestible component and at least 13% of dietary fiber and is up to 2 kcal/g in both caloric value 1 and caloric value 2.
3) When a greater proportion of glucose is removed from the hydrolyzate to obtain an indigestible fraction, the fraction contains at least 90% of an indigestible component and at least 20% of dietary fiber and is up to 1.8 kcal/g in caloric value 1 and up to 1.2 kcal/g in caloric value 2.
4) When disaccharides and oligosaccharides are removed from the hydrolyzate along with glucose, the resulting dextrin can be given a still higher dietary fiber content.

The present invention has been accomplished based on these novel findings.

Accordingly, the foregoing object of the present invention can be fulfilled by determining the structural requirement of the pyrodextrin to be used as the starting material of the invention and by providing an indigestible dextrin by hydrolyzing the pyrodextrin with alpha-amylase and glucoamylase and separating off a digestible fraction from the resulting hydrolyzate by ion exchange resin chromato-graphy.

DETAILED DESCRIPTION OF THE INVENTION

The values of analytical data as to samples (especially those of dextrin for use in the present invention) herein given are those calculated as solids. The number average molecular weight will be abbreviated as MN, the weight average molecular weight as MW, and the ratio of weight average molecular weight to number average molecular weight as MW/MN. Glucose residues having a $1 \rightarrow 4$ linkage only will be expressed as "glucose residues having a $1 \rightarrow 4$ linkage," and similar expressions will be used also for $1 \rightarrow 6$ linkage and $1 \rightarrow 3$ linkage. The numerical values used for the compositions of food examples and feed examples are those of moisture-containing components. The dietary fiber contents and caloric values given in these examples, except for those of indigestible dextrin, are calculated according to "Tables of Standard Compositions of Japanese Foods," Fourth Edition (edited by Science & Technology Agency, Resources Council, 1982).

The starch to be used for preparing the indigestible dextrin of the invention is corn starch, to which an acid needs to be used as a catalyst. Although various acids are usable, hydrochloric acid is especially preferable to use since the product is used for foods. To meet the requirement for use in foods, the product preferable has higher contents of indigestible component and dietary fiber. Thus, the product should contain at least 75% of an indigestible component and at least 13% of dietary fiber and should be up to 2.6 kcal/g in caloric value 1 and up to 2 kcal/g in caloric value 2. More preferably, the fraction of the product other than glucose should contain at least 90% of an indigestible component and at least 20% of dietary fiber and should be up to 1.8 kcal/g in caloric value 1 and up to 1.2 kcal/g in caloric value 2.

Incidentally, pyrodextrins include white dextrin which has heretofore been used generally for foods and pharmaceuticals. White dextrin contains up to 30% of indigestible component and up to 3% of dietary fiber, is about 3.9 kcal/g in both caloric values 1 and 2, and is therefore unusable for foods. Further when containing at least 30% of indigestible component and at least 12% of dietary fiber and having caloric values 1 and 2 of up to 3 kcal/g, such dextrin tastes stimulating and can not be used therefore.

The pyrodextrin for use in the present invention is prepared by adding an aqueous solution of hydrochloric acid, about 1% in concentration, to starch in an amount of 3 to 10% based on the starch, and heating the starch. Since the aqueous acid solution is added before the heat treatment, the starch and the acid are uniformly mixed together by being agitated and aged in a mixer, and the mixture is then heated at 150 to 200°C for 10 to 120 minutes, preferably for 15 to 60 minutes, unlike the heating condition for preparing conventional pyrodextrins (white dextrin and yellow dextrin). If the reaction temperature is higher, the resulting product will contain increased amount of indigestible component and dietary fiber and have a lower caloric value, whereas increased amount of colored substance will be formed as the temperature rises from about 180°C, so that the temperature is preferably 150 to 180°C.

Since the reaction can be conducted at a high temperature within a shortened period of time by suitably selecting the heater to be used, the mixture can be heat-treated efficiently using an apparatus capable of effecting a uniform reaction. Further because the starch is reacted in the form of powder, there arises a need to alter the heating condition for mass production, so that it is desirable to suitably vary the heating condition by checking the quality of the product as treated.

Next, the pyrodextrin is dissolved in water to a concentration of 20 to 45% and then hydrolyzed with alpha-amylase and thereafter with glucoamylase. Useful alpha-amylases are those commercially available, among which TERMAMYL (heat-resistant alpha-amylase produced by Bacillus licheniformis and manufactured by NOVO Industry Co., Ltd.) is most desirable.

Since the pyrodextrin solution has been made acidic with the acid added before the heat treatment, the solution must be adjusted to an optimum pH value for the amylase to be used, with any of common alkalis. Sodium hydroxide is commercially available in the form of a solution and is therefore most advantageous to use. The preferred pH is 5.5 to 6.5. If the value is lower than this range, a reduced reaction velocity will result, whereas higher pH values entail pronounced coloration. After the adjustment of pH, alpha-amylase is added to the solution usually in an amount of about 0.05 to about 0.2%.

The reaction temperature need not to be as high as that is used for the preparation of maltodextrin. Since high temperature rather results in promoted coloration, the temperature is preferably 80 to 90°C. Satisfactory results can be achieved by conducting the reaction usually for about 1 hour.

The reaction mixture is subsequently hydrolyzed with glucoamylase. Any of commercial glucoamylase is useful for this purpose. Glucoamylase generally contains a small amount of alpha-amylase, so that glucoamylase, if singly used, exhibits the effect to be produced by the conjoint use of alpha-amylase and glucoamylase. However, when the amount of alpha-amylase contained is lesser, the effect achieved is slightly smaller than is contemplated by the invention. The most preferred result can be achieved by using alpha-amylase and glucoamylase in combination. The pH preferable for the activity of glucoamylase is 4.0 to 6.0. Like alpha-amylase, glucoamylase is used in an amount of about 0.05 to about 0.2%. The reaction is conducted at a temperature of about 55 to about 60°C usually for 24 to 48 hours.

The amount of each of the amylases to be used is not limited to the foregoing range but may be used in an amount equivalent thereto in accordance with the activity of the amylase. The reaction time is controllable as desired by varying the amount.

The hydrolyzate obtained by hydrolyzing pyrodextrin with alpha-amylase may be autoclaved at 115 to 135°C, then reacted with alpha-amylase again and thereafter with glucoamylase, whereby the hydrolyzate can be filtered at a higher rate for refining.

The hydrolyzate resulting from the reaction with glucoamylase is lowered in pH to about 3.5, then heated to about 80°C, and thereafter decolorized with activated carbon, filtered, and desalted and decolorized with ion exchange resin in usual manner. The hydrolyzate is subsequently concentrated to a concentration of about 50% and thereafter subjected to continuous ion exchange resin chromatography to separate off the glucose formed. For this procedure, commercial strongly acidic cation exchange resins are usable.

Examples of such resins which are desirable are AMBERLITE IR-116,IR-118, IR120-B, XT-1022E and XT-471F (brand names for products of Japan Organo Co., Ltd.), DIAION 2K-1B, SKK-102, SK-104, SK-106,SK-110, SK-112, SK-116 and FR-01 (brand names for products of Mitsubishi Chemical Industries, Ltd.), and XFS-43281.00, -43280.00, -43279.00 and -43278.00 (brand names for products of Dow Chemical Japan).

It is desirable to use these resins usually as combined with an alkali metal or alkaline earth metal. To render the indigestible fraction efficiently separable from the glucose fraction, it is desirable to pass the hydrolyzate at a flow rate as adjusted to the resin used. The flow rate, SV, is 0.1 to 0.6, preferably 0.2 to 0.4. If the flow rate is outside this range, the operation efficiency or separation efficiency tends to become lower. The hydrolyzate is passed through the column at a temperature of 20 to 70°C, preferably 50 to 70°C. At lower temperature, inefficient separation will result, and an increase in the viscosity of the liquid is likely to cause trouble to the resin, whereas at higher temperature exceeding the specified range, the liquid is likely to turn brown or become otherwise degraded.

While the separation procedure decreases the glucose content to about 0.5%, the glucose content is adjustable as desired by altering the separation condition. Accordingly, in the case where it is desired to use the glucose as a sweetener, the product can be obtained with an increased glucose content. For example when the hydrolyzate as treated with glucoamylase has a glucose content of 50%, a product with an overall glucose content of about 33% can be obtained by separating off one-half, i.e., 25%, of the glucose from the hydrolyzate.

Further it is possible to separate off, along with glucose, a fraction including oligosaccharides and having a medium molecular weight by the separation procedure to obtain a fraction having an increased dietary fiber content of up to about 90%.

Experimental data will be described below to further clarify the features of the present invention.

EXPERIMENTAL EXAMPLES

1. Method of Determining the Content of Indigestible Component

The content was measured by a modified method according to "The method for determining the indigestible part using HPLC" (Journal of Japanese Society of Starch Science, Vol. 37, No. 2, p. 107, 1990) as will be described below.

One gram of a sample was accurately weighed out, and $50m\ell$ of 0.05M phosphate buffer (pH 6.0) was added to the sample, followed by addition of $0.1m\ell$ of Termamyl (alpha-amylase, product of NOVO Industry Co., Ltd.) and reacted at 95°C for 30 minutes. The reaction mixture was cooled and adjusted to a pH of 4.5. A $0.1m\ell$ quantity of amylo-glycosidase (product of Sigma) was added to and reacted with the mixture at 60°C for 30 minutes, followed by heating to 90°C to complete the reaction. The resulting mixture was diluted to $100m\ell$ with water, and the amount of glucose produced was determined by the pyranose-oxidase method. The content of indigestible component was calculated from the following equation.

$$\text{Content of indigestible component (\%)} = 100 - \text{Amount of glucose formed (\%)} \times 0.9$$

2. Method of Quantitatively Determining Glycosidic Linkages

A sample was methylated by a modified method of Hakomori's methylation method (S. Hakomori, J. Biochem., 55, 205(1964)) described below, followed by hydrolysis and thereafter by gas chromatography to quantitatively determine the glycosidic linkages composing the sample.

(1) Methylation

A dehydrated sample (100 to 200$\mu g$) is placed into a test tube (15$mm$ diam. $\times$ 100$mm$) with a screw cap and dissolved by addition of 0.3$m\ell$ of DMSO. To the solution is added 20$mg$ of NaH, immediately followed by addition of 0.1$m\ell$ of methyl iodide. The mixture is stirred by a touch mixer for 6 minutes and then cooled in ice water, and 2$m\ell$ of water is added to the mixture. The mixture is fully shaken with addition of 2$m\ell$ of chloroform. The upper layer (aqueous layer) is collected with a pipette and discarded. The remaining layer is similarly washed with addition of 2$m\ell$ of water. This procedure is repeated 6 times. Cotton is placed on the bottom of a Pasteur pipette, anhydrous sodium sulfate is placed into the pipette to form a 4- to 5-$cm$-thick layer, and the solution is passed through the layer for dehydration and then washed with chloroform. Subsequently, the solution is concentrated to dryness in a rotary evaporator.

(2) Hydrolysis

With addition of 0.5$m\ell$ of trifluoroacetic acid, the methylated product is hydrolyzed at 100°C for 4 hours, and the hydrolyzate is concentrated to dryness at 60°C in a rotary evaporator.

(3) Reduction

The hydrolyzate is dissolved in $0.5m\ell$ of water, and the solution is allowed to stand at room temperature for 2 hours with addition of $10mg$ of sodium borohydride. Several drops of acetic acid are added to the mixture until the mixture ceases foaming to terminate the reaction. The mixture is then dried at room temperature and further dried at room temperature with addition of $1m\ell$ of methanol to remove the boric acid formed. This procedure is repeated 6 times.

(4) Acetylation

With addition of $0.5m\ell$ or acetic anhydride, the reduced product is heated at 100°C for 4 hours and thereby acetylated. With addition of $1m\ell$ of toluene, the product is concentrated to dryness in a rotary evaporator.

(5) Desalting

The acetylated product is dissolved in $1m\ell$ of chloroform, the solution is shaken with addition of $1m\ell$ of water, and the aqueous layer is discarded. After repeating this procedure 5 times, the chloroform is evaporated off from the resulting layer by a rotary evaporator.

(6) Dissolving

The desalted product is dissolved in $0.5m\ell$ of chloroform and subjected to gas chromatography.

(7) Conditions for gas chromatography

| Column | DB-1 fused silica capillary column $60m \times 0.255m \times$ I.D., $0.1\mu m$ film |
|---|---|
| Column temperature | 50°C for 1 minute, elevation of temperature at a rate of 10°C/min to a constant temperature of 280°C |
| Temp. of sample vaporizing chamber | 300°C |
| Detection temp. | 300°C |
| Flow rate | $2.5m\ell$/min, helium |
| Detecting unit | flame ionization detector |

3. Method of Determining MN and MW

The same solution as used for the quantitative determination of glucose is passed through a column of ion exchange resin of the mixed bed type at SV of 1.0 for desalting, and the effluent is concentrated to e concentration of 5% using a rotary evaporator to obtain a sample. A 20-µl protion of the sample is subjected to liquid chromatography under the following conditions.

| Column | Shodex Ionpak S-802 · S-804 · S-805 · S-806 |
|---|---|
| Eluent | 1 ml/min of water |
| Column pressure | 40 $kg/cm^2$ |
| Column temp. | 60°C |
| Detector | RI |
| Data processor | Hitachi Model D-2000 GPC data processor |
| Standard sample | glucose, pullulan (with known molecular weight) |

MN and MW are calculated from the following equations based on the result of chromatography.

$$MN = \frac{\Sigma Hi}{\Sigma\ (Hi \div Mi)} \times QF$$

$$MW = \frac{\Sigma\ (Hi \times Mi)}{\Sigma\ Hi} \times QF$$

where

Hi: height of peak
Mi: molecular weight of pullulan
QF: Q factor (Mark-Houwink Coefficient)

4. Method of Quantitatively Determining Glucose

One gram of sample is accurately weighed out, placed into a 100-$m\ell$ measuring flask and diluted to 100$m\ell$ with distilled water. This solution is used for the quantitative determination of glucose by the pyranose oxidase (Determiner GL-E, product of Kyowa Medic Co., Ltd.) method.

5. Calculation of Physiological Combustion Heat Value

The physiological combustion heat value can be said a sum of the effective energies generated by the digestive absorption and the enzymatic absorption. Accordingly, the physiological combustion heat value can be calculated as follows:

The physiological combustion heat value (kcal/$g$)= (Starch A') $\times$ 4 + (Total amount of digestive absorption reducing sugars (B)) $\times$ 4 + (Total amount of digestive absorption sugar alcohol (C)) $\times$ 2.8 + (Total water-soluble sugars (A)) - (Total amount of digestive absorption reducing sugars (B) + Total amount of digestive absorption sugar alcohols (C) $\times$ 0.5)[1] $\times$ 1.9

6. Method of Measuring Caloric Value 1

According to the following method titled "Measurement of Physiological Combustion Heat of Food for Specified Health Use Containing Water-Soluble Low Calorie Sugars" which is notified by the Ministry of Health and Welfare of Japan.

7. Method of Measuring Caloric Value 2

The effective caloric value of a sample is calculated as the sum of the caloric value resulting from digestion and absorption by the digestive system up to the upper digestive tract, and the caloric value resulting from intestinal fermentation after arrival of the sample at the large intestine.

<u>Test 1</u>: Measurement of caloric value resulting from digestion and absorption by the upper digestive tract up to the small intestine

The sample is dissolved in 45mM (bis)Tris buffer (pH 6.0) containing 0.9mM calcium chloride to obtain a 4.55% solution, to which 160 U/$g$ of human saliva alpha-amylase (SIGMA Type IX-A) is added, followed by a reaction at 37°C for 30 minutes. After deactivating the enzyme, the reaction mixture is desalted with an ion exchange resin and adjusted to a concentration of 1.1%. The aqueous solution (4$m\ell$) is then added to 2$m\ell$ of 50mM hydrochloric acid-potassium chloride buffer (pH 2.0), and the mixture is maintained at 37°C for 100 minutes, followed by desalting with an ion exchange resin. To the desalted solution is added 45mM (bis)Tris buffer (pH 6.0) containing 0.9mM calcium chloride to adjust the solution to a concentration of 0.45%. To the solution is added 400U/$g$ of swine pancreatic amylase (product of Boehringer Mannheim Yamanouchi Co., Ltd.), followed by a reaction at 37°C for 6 hours. The enzyme is then deactivated, and the reaction mixture is thereafter desalted with an ion exchange resin, concentrated and lyophilized.

The powdery sample thus obtained is dissolved in 45mM sodium maleate buffer (pH 6.6) to prepare a 0.45% solution, with which 86U/$g$ of rat small intestine mucous membrane enzyme (product of SIGMA) is reacted at 37°C for 3 hours. The amount of glucose produced is measured by the pyranose oxidase method. The caloric value to be produced by digestion and absorption is calculated from the following equation.

$$\text{Caloric value} = \frac{\text{Amount of glucose produced (\%)} \times 4 \text{ kcal/}g}{100}$$

[1]: Ratio of Fermentable sugar of indigestible dextrin in the large intestines.

Test 2: Determination of caloric value resulting from intestinal fermentation

The caloric value of the fraction reaching the large intestine was determined by the growth curve method using rats as described below.

Table 1

| Component | Proportion(%) |
|---|---|
| Corn starch | 42.7 |
| Casein | 40.0 |
| Fiber | 2.0 |
| Mineral mixture | 10.0 |
| Vitamin mixture | 0.8 |
| DL-methionine | 0.3 |
| Choline bitartrate | 0.2 |
| Vegetable oil | 5.0 |

Rats were preliminarily raised for 5 days to adapt them to the laboratory environment and to the basal diet shown in Table 1, then checked for body weight and health and divided into groups (10 rats in each group). The average initial body weight of all the test groups was 79.6 to 80.8*g*. The body weight variations of the groups were in the range of 9 to 16*g*. The caloric value of all the test components and basal diet was measured by a bomb calorimeter.

Table 2

| No. | Basic diet (*g*) | Glucose (*g*) | Sample (*g*) | Total amount (*g*) | Caloric value (Kcal) |
|---|---|---|---|---|---|
| 1 | 5.4 | - | - | 5.4 | 22.7 |
| 2 | 5.4 | 0.5 | - | 5.9 | 24.7 |
| 3 | 5.4 | 1.0 | - | 6.4 | 26.7 |
| 4 | 5.4 | 2.0 | - | 7.4 | 30.7 |
| 5 | 5.4 | 4.0 | - | 9.4 | 38.7 |
| 6 | 5.4 | - | 0.5 | 5.9 | 24.7 |
| 7 | 5.4 | - | 1.0 | 6.4 | 26.7 |
| 8 | 5.4 | - | 2.0 | 7.4 | 30.7 |
| 9 | 5.4 | - | 4.0 | 9.4 | 38.7 |

After grouping, the rats were placed into individual steel cages and fed according to the experimental schedule listed in Table 2. The basal diet was given to all the rats in an amount of 5.4*g*/rat/*kg* (22.7 kcal/rat/day). For the test groups, glucose or the above sample was added in an amount of 0.5, 1.0, 2.0 or 4.0*g* to the basal diet. The amount of glucose or sample added was about 2, 4, 8 or 16 kcal/rat/day in terms of caloric value. The amount of ingestion was measured daily, and the gain in the body weight was measured on the 0th, 5th, 10th and 15th days. The rats were checked generally every day by observation.
Table 3 shows the results.

Table 3

| No. | Body weight | | | Consumed calories KCal/day | Weight gained (g) per calorie consumed |
|---|---|---|---|---|---|
| | Initial *g* | Final *g* | Gain *g*/14day | | |
| 1 | 79.2 | 74.4 | -4.8 | 23.2 | - |
| 2 | 79.9 | 78.9 | -1.0 | 24.8 | - |

Table 3   (continued)

| No. | Body weight | | | Consumed calories KCal/day | Weight gained (g) per calorie consumed |
|---|---|---|---|---|---|
| | Initial *g* | Final *g* | Gain *g*/14day | | |
| 3 | 79.4 | 85.5 | 6.1 | 26.2 | 0.017 |
| 4 | 79.5 | 89.9 | 10.4 | 27.6 | 0.027 |
| 5 | 79.6 | 96.2 | 16.6 | 28.9 | 0.041 |
| 6 | 79.2 | 78.3 | -0.9 | 24.2 | - |
| 7 | 79.4 | 77.8 | -1.6 | 26.9 | - |
| 8 | 79.8 | 82.8 | 3.0 | 28.3 | 0.008 |
| 9 | 79.5 | 86.3 | 6.8 | 29.6 | 0.016 |

With reference to Table 3, the caloric value determined by the animat experiment is:

$$(0.013 \div 0.023 \times 3.8 + 0.009 \div 0.051 \times 3.8) \div 2 = 1.41 \text{ kcal/}g$$

From Test 1, the caloric value resulting from the digestion and absorption of the sample by the upper digestive tract is:

$$\frac{9.8 \times 4 \text{ kcal/}g}{100} = 0.39 \text{ kcal/}g$$

Accordingly, the caloric value resulting from intestinal fermentation is:

$$1.41 - 0.39 = 1.02 \text{ kcal/}g$$

From this data, the caloric value produced by the intestinal fermentation of the dextrin is:

$$1.02 \div 0.912 \text{ (proportion reaching the large intestine)} = 1.1 \text{ kcal/}g = \text{about 1 kcal/}g$$

Thus, according to the methods of Test 1 and Test 2, the caloric value was calculated from the following equation.

$$\text{Caloric value 2 (kcal/}g) = \frac{\text{Glucose produced (\%)} \times 4}{100} + \frac{(100 - \text{glucose produced (\%))} \times 1}{100}$$

$$= 1 + \frac{3 \times \text{glucose produced (\%)}}{100}$$

Experimental Example 1

To 15*kg* of commercial corn starch was sprayed 1125*mℓ* of 1% hydrochloric acid solution, and the starch was treated in a mixer to prepare a uniform mixture. The mixture was placed into an aluminum vat, predried in a dryer at 120°C for 1 hour and then heat-treated at 165°C for 180 minutes. During the heat treatment, 2*kg* portions of the mixture were collected 10 minutes, 15 minutes, 30 minutes, 60 minutes, 120 minutes and 180 minutes after the start of the treatment to obtain six samples. The samples were analyzed to determine the contents of glucose, various glycosidic linkages, indigestible component and dietary fiber, caloric value 1, caloric value 2, MN and MW. Detected by this procedure were a glucose residue at each nonreducing end, glucose residues having a 1→ 4 linkage, glucose residues having a 1→ 6 linkage, glucose residues having a 1→ 3 linkage, glucose residues each having both 1→ 4 linkage and 1→ 6 linkage, glucose residues each having both 1→ 3 linkage and 1→ 4 linkage, glucose residue each having both 1→ 2 linkage and 1→ 4 linkage, and glucose residues having other linkages. The value of glucose determined by the method used included the content of glucose residues at the nonreducing ends, so that the content of glucose residues given is this value minus the content of glucose. Table 4 shows the values obtained.

The method of quantitative determination is complex and involves errors which are usually about ±5% and are invariably ±2% if minimum.

Table 4

| Item | | Heating time (min.) | | | | | |
|---|---|---|---|---|---|---|---|
| | | 0 | 15 | 30 | 60 | 120 | 180 |
| Content of Indigestible component (%) | | 55.9 | 58.8 | 68.1 | 71.6 | 73.9 | 74.7 |
| Contents of linkage (%) | non-reduced end | 18.2 | 19.5 | 19.9 | 19.4 | 18.9 | 19.8 |
| | 1→4 | 59.4 | 57.3 | 47.6 | 46.2 | 45.2 | 46.1 |
| | 1→6 | 5.2 | 5.9 | 9.0 | 9.7 | 9.7 | 10.5 |
| | 1→3 | 2.8 | 3.8 | 5.6 | 6.0 | 6.2 | 5.3 |
| | 1→4, 1→6 | 7.6 | 7.1 | 7.8 | 8.4 | 8.5 | 8.7 |
| | 1→3, 1→4 | 0.9 | 1.4 | 1.2 | 1.3 | 1.4 | 1.5 |
| | 1→2, 1→4 | 1.9 | 2.4 | 2.3 | 2.5 | 2.6 | 2.8 |
| | others | 4.0 | 2.6 | 6.6 | 6.5 | 7.5 | 5.3 |
| Content of dietary fiber (%) | | 10.8 | 13.9 | 24.5 | 29.1 | 31.3 | 32.8 |
| Caloric value 1 (kcal/g) | | 2.67 | 2.60 | 2.38 | 2.30 | 2.24 | 2.22 |
| Caloric value 2 (kcal/g) | | 2.32 | 2.24 | 1.96 | 1.85 | 1.78 | 1.76 |
| M N | | 1516 | 1302 | 1317 | 1449 | 1675 | 1668 |
| M w × 10^4 | | 57.3 | 40.2 | 49.3 | 157 | 272 | 395 |
| M w/M N | | 37.8 | 30.9 | 37.4 | 108 | 162 | 237 |

With the sample heated for 180 minutes, the prolonged heating presumable broke down the component sugars of starch, so that the results given in Table 4 will be discussed except for this sample. The contents of indigestible component and dietary fiber increase in proportion to the heating time. The caloric values decrease in inverse proportion to the heating time. The contents of glucose residues with various glycosidic linkages, i.e., those having 1→ 6 glycosidic linkage, those having 1→ 3 glycosidic linkage, those having both 1→ 4 and 1→ 6 glycosidic linkages, those having both 1→ 2 and 1→ 4 glycosidic linkages, and those having other linkages, increase in proportion to the heating time.

Only the content of residues with 1→ 4 linkage decreases in inverse proportion to the heating time. The values MN and MW/MN decrease during heating for 15 minutes and increase again in proportion to the heating time after 30 minutes. These variations in the glycosidic linkage contents and average molecular weights with the heating time are novel findings obtained by the experiment for the first time.

Experimental Example 2

2 liters of water was added to 1 *kg* of each of the six samples of Experimental Example 1 to prepare a solution, which was then adjusted to a pH of 6.0 with 20% sodium hydroxide and hydrolyzed with 0.2 wt. % of alpha-amylase (TERMAMYL 60L, product of NOVO Industry Co., Ltd.) at 85°C for 1 hour. The hydrolyzate was cooled to a temperature of 55°C, then adjusted to a pH of 5.5 and hydrolyzed with 0.2 wt.% of glucoamylase (product of Daiwa Kasei Co., Ltd.) for 36 hours, whereupon the resulting hydrolyzate was adjusted to a pH of 3.5 to inactivate the glucoamylase. The hydrolyzate was refined by decolorization with activated carbon, filtration and desalting with an ion exchange resin. The samples thus obtained were analyzed in the same manner as in Experimental Example 1. Table 5 shows the values obtained. The hydrolyzate obtained before the addition of glucoamylase was also checked for MN, MW and MW/MN. Table 6 shows the results.

Table 5

| Item | | 10 | 15 | 30 | 60 | 120 | 180 |
|------|---|-----|-----|-----|-----|-----|-----|
| | | **Heating time (min.)** | | | | | |
| Contents of linkage (%) | non-reduced end | 15.3 | 15.7 | 14.9 | 14.4 | 15.2 | 17.3 |
| | 1→4 | 16.4 | 16.5 | 17.7 | 18.4 | 18.0 | 18.8 |
| | 1→6 | 6.4 | 6.4 | 8.7 | 9.4 | 9.9 | 10.5 |
| | 1→3 | 4.8 | 5.6 | 7.3 | 7.6 | 7.5 | 8.1 |
| | 1→4, 1→6 | 5.7 | 5.7 | 6.5 | 7.1 | 7.3 | 7.7 |
| | 1→3, 1→4 | 1.0 | 1.0 | 1.2 | 1.3 | 1.3 | 1.4 |
| | 1→2, 1→4 | 1.5 | 1.5 | 2.1 | 2.3 | 2.3 | 2.5 |
| | others | 5.2 | 5.0 | 7.5 | 9.9 | 9.9 | 6.6 |
| Content of glucose (%) | | 43.7 | 42.6 | 34.1 | 29.6 | 28.6 | 27.1 |
| Content of indigestible component (%) | | 53.2 | 54.2 | 62.2 | 66.6 | 67.7 | 69.3 |
| Content of dietary fiber(%) | | 10.4 | 13.4 | 24.0 | 28.6 | 30.7 | 32.2 |
| Caloric value 1(kcal/g) | | 2.73 | 2.71 | 2.52 | 2.41 | 2.39 | 2.35 |
| Caloric value 2(kcal/g) | | 2.40 | 2.37 | 2.13 | 2.00 | 1.97 | 1.92 |
| M N | | 208 | 192 | 224 | 234 | 238 | 266 |
| M W× $10^{-3}$ | | 8.66 | 7.14 | 18.7 | 23.5 | 48.2 | 58.5 |
| M W/M N | | 41.6 | 37.2 | 83.4 | 100 | 203 | 220 |

Table 6

| Item | | Heating time (min.) | | | | | |
|------|---|-----|-----|-----|-----|-----|-----|
| | | 10 | 15 | 30 | 60 | 120 | 180 |
| MN | | 814 | 709 | 805 | 886 | 996 | 1005 |
| MW× $10^{-3}$ | | 29.7 | 23.1 | 28.1 | 89.3 | 168 | 286 |
| MW/MN | | 36.4 | 32.6 | 34.9 | 101 | 169 | 284 |

Table 5 reveals the following distinct features.

13

1) The 1→ 4 glycosidic linkage fraction greatly diminished, but about 16 to about 19% thereof still remained unhydrolyzed. This means that the 1→ 4 linkage fraction which should have been hydrolyzed almost completely with glucoamylase remained unhydrolyzed in an amount as large as 16 to 19%.

2) No marked hydrolysis occurred in the fractions except in the 1→ 4 glycosidic linkage fraction. The fact that the caloric values remained almost unincreased indicates that the low-calorie fraction remained almost unhydrolyzed with alpha-amylase and glucoamylase.

3) Suppose one-half of glucose is removed, for example, from the sample prepared by 10 minute heating. This results in a dietary fiber content of 13.3%.

4) The ratio MW/MN, which is 41.5 to 220, is exceedingly greater than the corresponding value of the prior art which is up to 20.

Table 6 reveals that the hydrolyzates before being hydrolyzed with glucoamylase were as high as about 36 to about 280 in MW/MN.

These result are novel findings obtained by the experiment for the first time.

Experimental Example 3

Each of the six samples of Experimental Example 2 was concentrated to obtain about 1.5 liters of 50% solution. 1 liter portion of the solution was passed, at a solution temperature of 60°C and at SV 0.25, through a column packed with 10 liters of XFS-43279.00 (product of Dow Chemical Japan), strongly acidic cation exchange resin of the alkali metal type. Subsequently, water was passed through the column to collect an indigestible fraction (as separated off from the glucose fraction). The sample thus obtained was analyzed in the same manner as in Experimental Example 1. Table 7 shows the results including average molecular weights, etc. In Table 7, the values were expressed in percentages based on the fraction other than glucose. The content (%) of indigestible component in the fraction other than glucose is a value obtained by subtracting the glucose content (%) from 100, dividing the measured amount of indigestible component by the remainder and multiplying the quotient by 100. Similarly, the content (%) of dietary fiber in the fraction other than glucose is a value obtained by subtracting the glucose content (%) from 100, dividing the measured amount of dietary fiber by the remainder and multiplying the quotient by 100.

Further similarly, the caloric value of the fraction other than glucose is a value obtained by multiplying the glucose content (%) by 4 (caloric value of 1$g$ of glucose), dividing the product by 100 and subtracting the quotient from the measured caloric value. The theoretical yield is a value obtained by subtracting the glucose content of Table 5 from 100.

## Table 7

| Item | Heating time (min.) | 10 | 15 | 30 | 60 | 120 | 180 |
|---|---|---|---|---|---|---|---|
| Content of non-reduced end | | 27.2 | 27.4 | 22.5 | 20.4 | 21.3 | 23.7 |
| Contents of linkage (%) | 1 → 4 | 29.1 | 28.8 | 26.9 | 26.1 | 25.2 | 25.8 |
| | 1 → 3 | 11.4 | 11.1 | 13.2 | 13.4 | 13.9 | 14.4 |
| | 1 → 5 | 8.5 | 9.8 | 11.1 | 10.8 | 10.5 | 11.1 |
| | 1 → 4 , 1 → 6 | 10.1 | 9.9 | 9.9 | 10.1 | 10.2 | 10.6 |
| | 1 → 3 , 1 → 4 | 1.8 | 1.7 | 1.8 | 1.8 | 1.8 | 1.9 |
| | 1 → 2 , 1 → 4 | 2.7 | 2.6 | 3.2 | 3.3 | 3.2 | 3.4 |
| | others | 9.2 | 8.7 | 11.4 | 14.1 | 13.9 | 9.1 |
| Content of glucose (%) | | 0.7 | 0.5 | 0.7 | 0.6 | 0.7 | 0.8 |
| Content of indigestible component (%) | | 94.5 | 98.3 | 97.2 | 97.1 | 97.2 | 97.0 |
| Content of dietary fiber (%) | | 21.1 | 24.4 | 37.4 | 41.7 | 44.1 | 45.1 |
| Caloric value 1 (kcal/g) | | 1.75 | 1.66 | 1.69 | 1.69 | 1.69 | 1.69 |
| Caloric value 2 (kcal/g) | | 1.17 | 1.05 | 1.08 | 1.09 | 1.08 | 1.09 |
| MN | | 995 | 1013 | 1148 | 1191 | 1281 | 1273 |
| MW × 10⁻³ | | 16.3 | 13.1 | 28.7 | 34.4 | 68.7 | 79.6 |
| MW/MN | | 16.4 | 12.9 | 25.0 | 28.9 | 54.4 | 62.5 |
| Theoretical yield (%) | | 56.3 | 57.4 | 65.9 | 70.4 | 71.4 | 72.9 |

With reference to Table 7, the content of indigestible component and the caloric values each remain substantially unchanged with the heating time, but the dietary fiber content increases in proportion to the heating time. The theoretical yield, which corresponds to the proportions of indigestible component, dietary fiber and low calorie component, increases in proportion to MN, MW and MW/MN. The table further reveals that the theoretical yield increases to at least about 65% when MW/MN is at least 25. This indicates that the hydrolyzate before the separation of the glucose fraction by the ion exchange resin is high in the contents of indigestible component and dietary fiber and low in caloric values.

(Incidentally, the content of indigestible component in the overall hydrolyzate containing glucose can be readily obtained by subtracting the glucose content (%) from 100, multiplying the corresponding content of Table 7 by the remainder and dividing the product by 100. Similarly, the content of dietary fiber in the overall hydrolyzate containing glucose can be obtained by subtracting the glucose content (%) from 100, multiplying the corresponding content of Table 7 by the remainder and dividing the product by 100. Further the caloric value of the overall hydrolyzate containing glucose can be obtained by multiplying the glucose content (%) by 4, dividing the product by 100 and adding the quotient to the caloric value of Table 7.) The relationship between the important value MN and the different glycosidic linkage fractions was investigated by regression analysis for determining the correlation between variables to obtain equations and correlation coefficients. The correlation analysis was conducted for the five samples except the sample which was obtained by 180 minutes heating and wherein the component sugars appeared to have been broken down, using the amounts of glucose residues having various glycosidic linkages as predictor variables and the MN values as criterion variables. Table 8 shows eight equations and correlation coefficients obtained.

$$Y = A0 + An·Xn$$

Where

Y :  MN of the component other than glucose
X1:  amount (%) of nonreducing end glucose residues
X2:  amount (%) of glucose residues having a 1→ 4 glycosidic linkage
X3:  amount (%) of glucose residues having a 1→ 6 glycosidic linkage
X4:  amount (%) of glucose residues having a 1→ 3 glycosidic linkage
X5:  amount (%) of glucose residues having 1→ 4 and 1→ 6 glycosidic linkages
X6:  amount (%) of glucose residues having 1→ 3 and 1→ 4 glycosidic linkages
X7:  amount (%) of glucose residues having 1→ 2 and 1→ 4 glycosidic linkages
X8:  amount (%) of glucose zesidues having other glycosidic linkages

Table 8

| No. | equation | correlation coefficient |
|-----|----------|------------------------|
| 1 | Y=1930-33.841·X1 | 0.927 |
| 2 | Y=3067-71.309·X2 | 0.997 |
| 3 | Y=-49+93.01·X3 | 0.970 |
| 4 | Y=244+86.924·X4 | 0.744 |
| 5 | Y=-3555+466.224·X5 | 0.517 |
| 6 | Y=-1380+1407.91·X6 | 0.520 |
| 7 | Y=130+331.904·X7 | 0.888 |
| 8 | Y=609+45.081·X8 | 0.943 |

Consequently, MN was found to have the highest correlation with X2 (amount of glucose residues having a 1→ 4 glycosidic linkage) of all the contents of eight kinds of glycosidic linkages as represented by the equation of Table 8, No. 2 (correlation coefficient: 0.997). This equation (hereinafter referred to as "Equation 1") reveals the novel fact that the smaller the amount of glucose residue having a 1→ 4 glycosidic linkage, the greater the MN, that is, the higher the content of indigestible component and dietary fiber the lower the caloric value.

Experimental Example 4

To 300$kg$ of commercial corn starch was added 5.8 liters of 3% hydrochloric acid, and the starch was treated in the same manner as in Experimental Example 1 except that the starch was heat-treated at 180°C for 30 minutes, followed by the same procedures as in Experimental Examples 2 and 3 to obtain a sample. The sample was analyzed in the same manner as in Experimental Example 3.

Experimental Example 5

To 300$kg$ of commercial corn starch was added 9 liters of 2% hydrochloric acid, and the starch was treated in the same manner as in Experimental Example 1 except that the starch was heat-treated at 150°C for 60 minutes, followed by the same procedure as in Experimental Example 4 to obtain a sample, which was then analyzed in the same manner as in Experimental Example 3. Table 9 shows the analytical results obtained in Experimental Examples 4 and 5 and including MN values which are given in comparison with those calculated from Equation 1.

Table 9

| Item | | Example | |
|---|---|---|---|
| | | 4 | 5 |
| Contents of linkage (%) | non-reduced end | 23.6 | 25.0 |
| | $1 \to 4$ | 27.4 | 26.6 |
| | $1 \to 6$ | 12.9 | 12.6 |
| | $1 \to 3$ | 10.4 | 10.3 |
| | $1 \to 4, 1 \to 6$ | 9.8 | 9.9 |
| | $1 \to 3, 1 \to 4$ | 1.7 | 1.8 |
| | $1 \to 2, 1 \to 4$ | 2.9 | 3.0 |
| | others | 11.3 | 10.8 |
| content of glucose (%) | | 0.5 | 0.7 |
| content of indigestible component (%) | | 97.7 | 96.3 |
| content of dietary fiber(%) | | 42.0 | 44.5 |
| caloric value 1(kcal/$g$) | | 1.68 | 1.71 |
| caloric value 2(kcal/$g$) | | 1.07 | 1.11 |
| MN measured value | | 1161 | 1084 |
| MN culculated value | | 1113 | 1170 |
| Difference between calculated value and measurement (%) | | -4.1 | +7.9 |

The Variation of the calculated value from the measured value was -4.1% in Experimental Example 4 and +7.9% in Experimental Example 5.

Experimental Example 6

The sample of Experimental Example 2 obtained by 30 minutes heating was concentrated to prepare about 1.5 liters of 50% solution. The solution (100$m\ell$) was passed through a column packed with 160$m\ell$ of Ionpack S-2006 (product of Showa Denko Co., Ltd.) which is a styrene-divinylbenzene copolymer of the sodium type having its molecular weight corrected with pullulan, at a column temperature of 60°C and at SV of 0.25. Subsequently, water was passed through the column to collect four fractions (as separated from a fraction of glucose and oligosaccharides). The four fractions were checked for the content of dietary fiber. Table 10 shows the result.

Table 10

| | Sample No. | | | |
|---|---|---|---|---|
| | 1 | 2 | 3 | 4 |
| content of dietary fiber (%) | 90.3 | 51.5 | 44.2 | 23.4 |

The dietary fiber content, which was 37.4% in Table 7, increased to a maximum of 90.3% as shown in Table 10.

Comparative Example 1

A sample was prepared in the same manner as in Experimental Example 4 with the exception of adding 22.5 liters of 1% hydrochloric acid to 300$kg$ of commercial potato starch, treating the mixture in the same manner as in Experimental Example 1 and heating the mixture at 165°C for 1 hour. The sample was analyzed in the same manner as in Experimental Example 4. MN was calculated from Equation 1.

## Comparative Example 2

A sample was prepared in the same manner as in Experimental Example 4 with the exception of adding 22.5 liters of 1% hydrochloric acid to 300*kg* of commercial tapioca starch, treating the mixture in the same manner as in 1 Experimental Example and heating the mixture at 165°C for 1 hour. The sample was analyzed in the same manner as in Experimental Example 4, and MN was calculated from Equation 1.

Table 11 shows the results obtained in Comparative Examples 1 and 2.

Table 11

| Item | | Comp. Ex. | |
|---|---|---|---|
| | | 1 | 2 |
| Contents of linkage (%) | non-reduced end | 28.6 | 23.9 |
| | 1→ 4 | 30.0 | 25.0 |
| | 1→ 6 | 11.7 | 13.9 |
| | 1→ 3 | 9.6 | 10.8 |
| | 1→ 4, 1→ 6 | 10.4 | 9.5 |
| | 1→ 3, 1→ 4 | 1.8 | 1.5 |
| | 1→ 2, 1→ 4 | 2.7 | 2.9 |
| | others | 5.2 | 12.5 |
| content of glucose (%) | | 1.2 | 1.2 |
| content of indigestible component (%) | | 97.3 | 94.3 |
| content of dietary fiber(%) | | 25.1 | 33.9 |
| caloric value 1(kcal/*g*) | | 1.68 | 1.75 |
| caloric value 2(kcal/*g*) | | 1.08 | 1.17 |
| MN measured value | | 750 | 1119 |
| MN calculated value | | 928 | 1363 |
| Difference between calculated value and measurement (%) | | +23.7 | +21.9 |

With reference to Table 11, the difference of the calculated MN value from the measured value is +23.7% in Comparative Example 1 and +21.8% in Comparative Example 2, hence a great difference. This indicates that the correlation between the 1→ 4 glycosidic linkage content and MN as represented by Equation is absent therebetween, showing that different starches serving as materials produce products which are greatly different in structure even if heat-treated under the same condition.

## Experimental Example 6-2

The eight pyrodextrin samples obtained in Experimental Examples 1, 4 and 5 were checked for the degree of coloration by measuring the whiteness of the samples relative to the whiteness of magnesium oxide taken as 100%, using photoelectric whiteness meter (product of Kett Co.) and a blue filter. Table 12 shows the results.

Table 12

| Ex. | heating temp. (°C) | heating time (min.) | Whiteness (%) |
|---|---|---|---|
| 1 | 165 | 10 | 72.3 |
| 1 | 165 | 15 | 68.2 |
| 1 | 165 | 30 | 47.9 |
| 1 | 165 | 60 | 38.5 |
| 1 | 165 | 120 | 33.8 |

Table 12 (continued)

| Ex. | heating temp. (°C) | heating time (min.) | Whiteness (%) |
|---|---|---|---|
| 1 | 165 | 180 | 30.8 |
| 4 | 180 | 30 | 46.8 |
| 5 | 150 | 60 | 49.4 |

Table 12 shows that the whiteness decreases in inverse proportion to the heating time and heating temperature.

Next to check the indigestible dextrin of the invention for physiological activities, the samples of Examples 1 and 3 to be described later were used, which will be referred to as "samples A and B," respectively, in Experimental Examples 7 to 11.

Experimental Example 7

Clinical test

The sample A was given at a daily dose of 10$g$ to ten males in good health during a test period of 2 weeks. During the first and second weeks of the test period, they are given the same meals in same quantities, and the sample was administered after breakfast on Monday through Friday. Feces were collected on every defecation and checked for wet weight, dry weight, moisture content and frequency of defecation. Table 13 shows the results, which reveal that the sample has an effect to increase the overall amount of feces.

Table 13

| Test period | Non-ingestion period | Ingestion period |
|---|---|---|
| Wet weight of feces | 535 | 782 * |
| Dry weight of feces | 111 | 166 * |
| Amount of water in feces | 430 | 620 * |
| Water content of feces | 76.5 | 77.7 |
| Frequency of excretion | 4.5 | 5.8 * |

Each value listed is mean value ± standard deviation, and the mark * indicates a significance level of 5% relative to the non-ingestion period, hence a significant difference.

Experimental Example 8

Clinical test

The sample B was checked for a constipation alleviating effect. The, sample was given to 25 volunteers having a tendency toward constipation at a predetermined dose for at least 5 days. Changes resulting from the administration of the sample in defecation were checked with a questionnaire. Scores were assigned to the check items on the questionnaire according to the following criteria to substantiate the effect through a statistical procedure.

| (1) Frequency of excretion | |
|---|---|
| At least once/day | score 4 |
| Once/day | score 3 |
| Once/two days | score 2 |
| Once/three days | score 1 |
| (2) Amount of excretion | |
| Large | score 4 |
| Usual | score 3 |

(continued)

| (1) Frequency of excretion | |
|---|---|
| (2) Amount of excretion | |
| Small | score 2 |
| None | score 1 |
| (3) State of feces | |
| Bananalike, pasty | score 2 |
| Hard | score 1 |
| (4) Feeling after defecation | |
| Complete discharge | score 2 |
| Incomplete discharge | score 1 |

Table 14 shows the results. The mark * listed indicates a significance level of 5% relative to "before administration," hence a significant difference.

Table 14

| | Amount administered | |
|---|---|---|
| | 5g | 10g |
| Before administration | 8.44 | 7.20 |
| After administration | 10.60 * | 11.80 * |

With reference to Table 14, the sample 3, when administered at a dose of at least 5g, resulted in increased scores and was found effective for alleviating constipation.

Experimental Example 9

Animal experiment

Male rats of Sprague-Dawley strain (6 rats/group) initially weighing about 50g were accommodated in individual cages placed in a small animal breeding chanber controlled to 23 ± 2°C, preliminarily raised with a commercial synthetic diet for 1 week, and thereafter fed for 7 days with a basal diet shown in Table 15, or the basal diet containing 5% of the sample A added thereto, or the basal diet containing 5% of cellulose ( Avicel, product of Sanyo-Kokusaku Pulp Co., Ltd.) with free access to water and the diet. The intake of diet and change in body weight were recorded daily. On the seventh day, the animals were sacrificed with blood taken, and the cecum was removed and checked for the weight thereof, pH of the contents of the cecum and the amount of butyric acid therein. Table 16 shows the results obtained. The mark * in Table 16 indicates a significance level of 5%, hence a significant difference.

Table 15

| Material | Weight parts |
|---|---|
| Casein | 25 |
| Corn oil | 5 |
| Salt (MM-2) mixture | 4 |
| Vitamin (Harper) mixture | 1 |
| Choline chloride | 0.2 |
| Vitamin E | 0.05 |
| Sucrose | 64.75 |

Table 16

| | Weight of cecum (g) | Contents of cecum (pH) | Amount of butyric acid (mg/cecum) |
|---|---|---|---|
| Basal diet | 1.2 | 7.6 | 4.0 |
| Basal diet+ Sample A | 4.0 * | 5.8 * | 21.0 * |
| Basal diet+ cellulose | 1.5 | 7.5 | 4.0 |

The results listed in Table 16 reveal that the sample A reached the large intestine while remaining indigested, metabolized to organic acids under the action of enterobacteria and resulted in a lower pH within the intestine.

Experimental Example 10

Animal test

Rats were used for a nutritional experiment to check the samples A and B for a serum lipid lowering effect.
Male rats of Sprague-Dawley strain initially weighing about 50g (3 week old, provided by CLEA Japan Co.) were preliminarily raised for two weeks on a high-sucrose diet (basal diet) shown in Table 15, and thereafter raised and divided into three groups (16 rats in each group) for 9 weeks, during which the basal diet was given to the first group (control group), and a test diet comprising 95 % of the basal diet and 5 % of the sample A or B admixed therewith was given to the second group (sample A group) and the third group (sample B group), with free access to the diet and water. In the 9th week, the rats were fasted for 4 hours, blood was then taken, and the serum total cholesterol value and neutral fat value thereof were determined by a kit for enzyme method (product of Wako Junyaku Co., Ltd.). Table 17 shows the results. The mark ∗ in Table 17 indicates a significance level of 5%, hence a significant difference.

Table 17

| Item | Group | | |
|---|---|---|---|
| | Comp. | Sample A | Sample B |
| Weight gain (g/9weeks) | 298 | 276 | 310 |
| Diet efficiency Serum total | 0.22 | 0.22 | 0.22 |
| cholesterol (mg/dl) | 99 | 64 * | 80 * |
| Serum neutral fat (mg/dl) | 275 | 129 * | 177 * |

The results achieved by the test groups are expressed in mean values. The diet efficiency was calculated from Equation 2.

Equation 2 :

$$\text{Diet efficiency} = \text{weight gain} \div \text{diet intake}$$

As seen from Table 17, there was no difference between the three sample groups in weight gain and diet effecieny. However, the samples A and B taking groups were apparently lower in serum total cholesterol value and neutral fat value than the control group. The samples A and B were found to be effective, and the sample A was slightly superior to the sample B.

Experimental Example 11

Clinical test

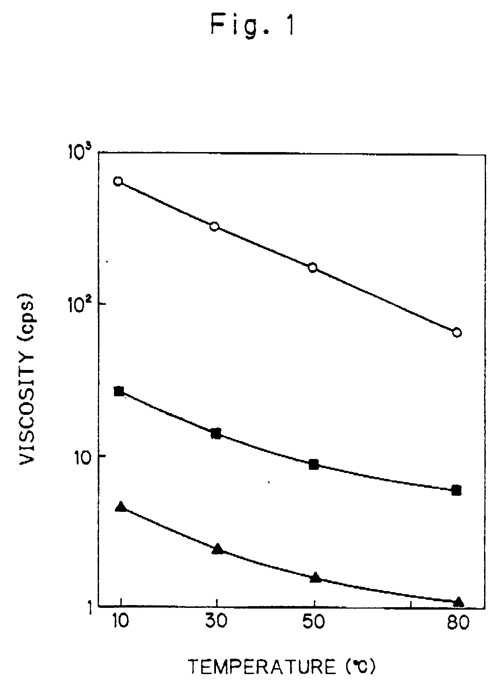

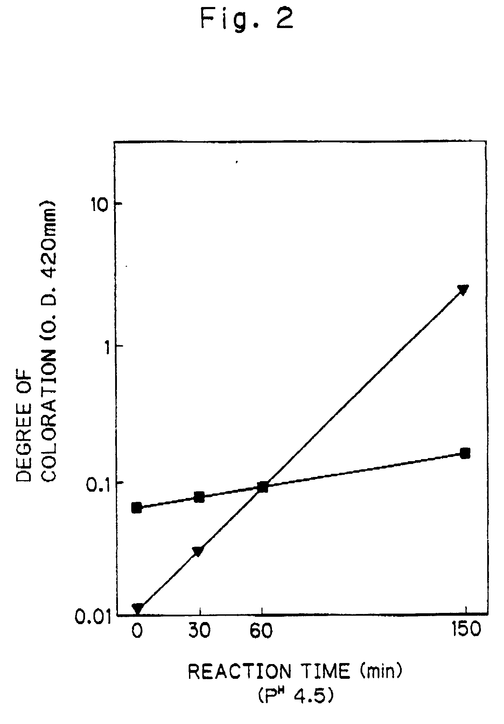

The sample A (10g) was dissolved in 100mℓ of water, and the solution was orally given to 10 persons three times a day before every meal for two months, during which they observed usual eating habit and were allowed to perform routine work. The persons participating in the test were 33 to 59 years old (50.3 in average age), 158 to 173*cm* tall (164.8*cm* on the average) and weighed 52 to 82*kg* (68.8*kg* on the average). FIG. 1 and FIG. 2 shows the results obtained.

FIG. 1 and FIG 2 reveal that the administration of the sample A altered the serum total cholesterol value toward the normal value (120-250 *mg/dℓ*). Those who were higher than the normal in this value exhibited a reduction, while those who were lower than the normal exhibited a value in the normal range. A similar result was observed also with respect to the neutral fat value. These results substantiate that the sample A has a remarkable effect improving serum lipid metabolism.

Summary of Results of Experimental Data Analysis

To sum up the foregoing results of experimental data analysis, the product of the invention obtained by hydrolyzing pyrodextrin with alpha-amylase and glucoamylase distinctly differs from known pyrodextrins with respect to the following. More specifically stated, the fraction of the present dextrin other than glucose has the following features.

(1) The content of indigestible component is max 98.3%, and the dietary fiber content is max 45.1%. With the fraction of oligosaccharides further removed, the fraction maximally contains 90.3% of dietary fiber and is minimally 1.66 kcal/g in caloric value 1 and 1.05 kcal/g in caloric value 2.

(2) In MN, the fraction is about 990 to about 1300 as compared with conventional pyrodextrins which are at least 1300. The theoretical yield is at least about 65% in the case where MW/MN is at least 25. The contents of indigestible component and dietary fiber increase in proportion to the value MW/MN, and the caloric value decreases in inverse proportion thereto.

(3) In the content of glucose residues having 1→ 4 glycosidic linkage, the fraction is about 25 to about 30% against about 57% of known pyrodextrins.

(4) In the content of glucose residues having 1→ 6 glycosidic linkage, the fraction is about 11 to about 14% against about up to 3% of known pyrodextrins.

(5) In the content of glucose residues having 1→ 3 glycosidic linkage, the fraction is about 8 to about 11% against about up to 1% of known pyrodextrins.

(6) In the content of glucose residues having other glycosidic linkages, the fraction is about 9 to about 14% against about 20% of known pyrodextrins.

(7) As represented by Equation 1, the content of glucose residues having 1→ 4 glycosidic linkage has close correlation with the MN of the fraction. This means that there is close correlation between the content of glucose residues having 1→ 4 glycosidic linkage and the proportions of indigestible component and dietary fiber formed.

(8) A starch other than corn starch, i.e., potato starch or tapioca starch, was treated under the same condition as corn starch. The content of glucose residue having 1→ 4 glycosidic linkage and present in the product obtained was substituted in Equation 1 to calculate MN. A great difference of at least about 22% was found between the calculated value and the actual measurement. This indicates that Equation 1 is true only with corn starch specifically.

(9) An increase in the content of indigestible component results in an increased dietary fiber content and a reduced caloric value.

(10) Furthermore, the indigestible dextrin, when ingested, is found effective for improving the internal environment of the intestine and eliminating constipation and diarrhea as by giving a lower pH to the interior of the intestine and increasing the amounts of short chain fatty acids having an intestine conditioning effect.

(11) The dextrin further acts to diminish cholesterol and neutral fats among other serum lipids, consequently preventing arterial sclerosis and hypertension.

(12) Because of these effects, the indigestible dextrin of the present invention is very useful as a material for alimentotherapy to accomplish the above purposes.

The above experimental results show that the product of the invention is a novel substance containing exceedingly larger amounts of indigestible component and dietary fiber and having a lower caloric value than conventional pyrodextrins, and greatly different from known pyrodextrins in structure.

The Experimental data further indicates that the whiteness decreases in inverse proportion to the heating time. The decrease in whiteness demonstrates an increase in the amount of colored substance due to the heat treatment. The increase in the amount of colored substance presents difficulty in refining the product before the separation, consequently lowering the efficiency of the ion exchange resin for use in the separation treatment. The whiteness must therefore be at least 30%, preferably at least 40%. Table 12 shows that the heat treatment is to be conducted preferably for not more than 60 minutes when the heating temperature is 150°C, or for not longer than about 45 minutes at 165°C or for not longer than 30 minutes at 180°C.

Although the progress of the reaction can be controlled by varying the amount of acid to be added to corn starch, use of a greatly increased amount of acid causes corrosion or abrasion to the apparatus, so that the optimum amount of acid to be used is up to 3000ppm, preferably about 1000ppm, based on the starch.

EXAMPLES

Examples of the present invention will be given below.

Example 1

Commercial corn starch (2500*kg*) was placed into a ribbon mixer, and 188 liters of 1% hydrochloric acid solution was sprayed onto the starch with use of pressurized air while rotating the mixer. The mixture was then passed through a disintegrator to obtain a uniform mixture, which was thereafter aged in the ribbon mixer for 8 hours. The mixture was predried to a moisture content of about 4% by a flash dryer, subsequently continuously charged into a converter of the rotary kiln type and heat-treated at 165°C for 40 minutes to obtain a pyrodextrin.

Water (4000 liters) was added to the pyrodextrin (2000*kg*) to prepare a solution, which was then adjusted to a pH of 6.0 with 20% aqueous solution of sodium hydroxide. With addition of 0.1 wt. % of alpha-amylase (TERMAMYL 60L, product of NOVO Industry Co., Ltd.), the solution was hydrolyzed at 90°C for 1 hour. The hydrolyzate was then autoclaved at 125°C for 10 minutes, thereafter discharged into the atmosphere, cooled to a temperature of 57°C, adjusted to a pH of 5.5 and hydrolyzed for 40 hours with 0.1 wt. % of glucoamylase (product of Daiwa Kasei Co., Ltd.) added thereto. The resulting hydrolyzate was adjusted to a pH of 3.6 to inactivate the glucoamylaze. The hydrolyzate was decolorized with activated carbon, filtered, desalted with ion exchange resins and thereafter concentrated to obtain a 50% solution. A 20 liter portion of the solution was passed at 60°C at SV 0.25 through the column of a continuous chromatographic device packed with 10 liters of XFS-43279.00 (product of Dow Chemical Japan), which is a strongly acidic cation exchange resin of the sodium type. Subsequently, water was passed through the column to separate off a glucose fraction and obtain an indigestible fraction. The fraction was concentrated and spray-dried to obtain about 5*kg* of an indigestible dextrin having a moisture content of 4.4%.

Example 2

Commercial corn starch (2500*kg*) was placed into a ribbon mixer, and 125 liters of 2% hydrochloric acid solution was sprayed onto the starch with use of pressurized air while rotating the mixer. The mixture was then passed through a disintegrator to obtain a uniform mixture, which was thereafter aged in the ribbon mixer for 10 hours. The mixture was predried to a moisture content of about 3% by a flash drier, subsequently continuously charged into a converter of the rotary kiln type and heat-treated at 150°C for 55 minutes to obtain a pyrodextrin.

Water (3000 liters) was added to the pyrodextrin (2000 *kg*) to prepare a solution, which was then adjusted to a pH 6.0 with 20% aqueous solution of sodium hydroxide. With addition of 0.2 wt. % of alpha-amylase (TERMAMYL 60L, product of NOVO Industry Co., Ltd.), the solution was hydrolyzed at 85°C for 40 minutes. The hydrolyzate was then autoclaved at 130°C for 10 minutes, thereafter discharged into the atmosphere, cooled to 86°C and hydrolyzed for 20 minutes with 0.05 wt. % of alpha-amylase added thereto. The resulting hydrolyzate was cooled to a temperature of 55t, adjusted to a pH of 5.5 and hydrolyzed for 36 hours with 0.2 wt. % of glucoamylase (product of Daiwa Kasei Co., Ltd.) added thereto, whereupon the hydrolyzate was adjusted to a pH of 3.5 to inactivate the glucoamylaze. The hydrolyzate was refined in the same manner as in Example 1 and further treated in the same manner as in Example 1 with the exception of using potassium-type AMBERLITE IR-118 (product of Japan Organo Co., Ltd.) as a strongly acidic ion exchange resin, whereby an indigestible fraction was obtained. This fraction Was concentrated to a concentration of 50% and then spray-dried, affording about 4.5 *kg* of an indigestible dextrin having a moisture content of 4.2%

Example 3

Commercial corn starch (2500*kg*) was placed into a ribbon mixer, and 100 liters of 3% hydrochloric acid solution was sprayed onto the starch with use of pressurized air while rotating the mixer. The mixture was then passed through a disintegrator to obtain a uniform mixture, which was thereafter aged in the ribbon mixer for 10 hours. The mixture was predried to a moisture content of about 3% by a flash drier, subsequently continuously charged into a converter of the rotary kiln type and heat-treated at 180°C for 25 minutes to obtain a pydrodextin.

Water (5000 liters) was added to the pydrodextrin (2000*kg*) to prepare a solution, which was then adjusted to a pH of 5.8 with 20% aqueous solution of sodium hydroxide. With addition of 0.15 wt. % of alpha-amylase (TERMAMYL 60L, product of NOVO Industry Co., Ltd.), the solution was hydrolyzed at 86°C for 1 hour. The hydrolyzate was then cooled to a temperature of 55°C, adjusted to a pH of 5.6 and hydrolyzed for 36 hours with 0.1 wt. % of glucoamylaze (product of Daiwa Kasei Co., Ltd.) added thereto. The resulting hydrolyzate was adjusted to a pH of 3.5 to inactivate the glucoamylase. The hydrolyzate was thereafter treated in the same manner as in Example 2, giving about 4*kg* of an indigestible dextrin having a moisture content of 4.8%.

Example 4

Commercial corn starch (2500*kg*) was placed into a ribbon mixer, and 376 liters of 0.5% hydrochloric acid solution was sprayed onto the starch with use of pressurized air while rotating the mixer. The mixture was then passed through a disintegrator to obtain a uniform mixture, which was thereafter aged in the ribbon mixer for 8 hours. The mixture was predried to a moisture content of about 4% by a flash drier, subsequently continuously charged into a converter of the rotary kiln type and heat-treated at 165°C for 15 minutes to obtain a pyrodextrin. Water (4000 liters) was added to the pyrodextrin (2000 *kg*) to prepare a solution, which was then adjusted to a pH of 6.0 with 20% aqueous solution of sodium hydroxide. With addition of 0.1 wt. % of alpha-amylase (TERMAMYL 60L, product of NOVO Industry Co., Ltd.), the solution was hydrolyzed at 82°C for 1 hour. The hydrolyzate was then autoclaved at 125°C for 10 minutes, thereafter discharged into the atmosphere, cooled to a temperature of 57°C, adjusted to a pH 5.5 and hydrolyzed for 36 hours with 0.1 wt.% of glucoamylase (product of Daiwa Kasei Co., Ltd.) added thereto. The resulting hydrolyzate was adjusted to a pH of 3.6 to inactivate the glucoamylase. The hydrolyzate was refined and concentrated in the same manner as in Example 1 to obtain a 52% solution. A 20 liter portion of the solution was passed at 60°C at SV 0.3 through the column of a continuous chromatographic device packed with 10 liters of DIAION SKK-116 (product of Mitsubishi Chemical Industries, Ltd.), which is a strongly acidic cation exchange resin of the sodium type. Subsequently, water was passed through the column to separate off 52% of glucose formed and obtain an indigestible fraction. The fraction was concentrated to obtain about 8*kg* of a liquid indigestible dextrin having a concentration of 70%.

The solutions obtained in Examples 1 to 4 before the separation procedure were checked for glucose content, and the indigestible dextrins obtained in these examples were analyzed to determine the glucose content, amount (%) of glucose removed, contents of various glycosidic linkages (determined by "Hakomori's methylation method") and content of indigestible component. The fraction of each of these dextrins other than glucose was also analyzed to determine the indigestible component, MN as actually measured, MN as calculated from Equation 1, variation of the calculated value from the measured value and MW/MN. The results are collectively listed in Table 18, which also shows the whiteness of each pyrodextrin.

Although, the appearent glucose separation persentages in these four examples are 97.5%, 87.8% 79.3% and 51.1%, respectively.

Table 18

| Item | | Example 1 | Example 2 | Example 3 | Example 4 |
|---|---|---|---|---|---|
| Contents of linkage (%) | 1→4 non-reduced end | 21.8 | 21.8 | 27.0 | 27.4 |
| | 1→6 | 27.8 | 26.6 | 28.5 | 27.5 |
| | 1→3 | 12.9 | 13.1 | 10.9 | 11.6 |
| | 1→4, 1→6 | 10.7 | 11.0 | 10.4 | 10.1 |
| | 1→3, 1→4 | 9.9 | 9.9 | 9.6 | 9.6 |
| | 1→2, 1→4 | 1.8 | 1.6 | 1.8 | 1.7 |
| | others | 3.1 | 3.2 | 3.2 | 3.2 |
| Content of glucose before separation (%) | | 12.0 | 12.8 | 8.6 | 8.9 |
| Content of glucose after separation (%) | | 0.8 | 4.7 | 8.4 | 20.4 |
| Removal amount of glucose (%) | | 97.5 | 87.3 | 79.3 | 51.1 |
| Content of indigestible component (%) | | 96.9 | 92.2 | 87.0 | 77.5 |
| Component of indigestible portion other than glucose (%) | | 97.7 | 96.7 | 95.0 | 97.3 |
| Content of dietary fiber (%) | | 31.7 | 38.6 | 40.5 | 41.7 |
| Component of dietary fiber other than glucose (%) | | 39.8 | 36.6 | 33.3 | 28.5 |
| Caloric value 1 (kcal/g) | | 40.1 | 38.4 | 36.4 | 35.8 |
| Caloric value other than glucose | | 1.71 | 1.89 | 2.08 | 2.50 |
| Caloric value 2 (kcal/g) | | 1.68 | 1.70 | 1.74 | 1.68 |
| Caloric value other than glucose | | 1.10 | 1.28 | 1.49 | 1.90 |
| Calory other than glucose | | 1.07 | 1.10 | 1.15 | 1.08 |
| MN measured value | | 1156 | 1088 | 1034 | 1021 |
| MN calculated value | | 1085 | 1170 | 1035 | 1106 |
| Difference between calculated value and measurement (%) | | -6.1 | +7.5 | +0.1 | +8.3 |
| MW/MN | | 26.2 | 39.8 | 28.6 | 35.8 |
| Whiteness (%) | | 43.8 | 46.7 | 50.1 | 57.2 |

The variations of the calculated values from the measured values were in the range of +8.3% to - 6.1%.

The indigestible dextrin is usable for almost all foods. The term "foods" as used herein refers collectively to foods for man and feeds for livestock and for use in zoos and for pets. The indigestible dextrin is prepared from starch, is soluble in water, contains dietary fiber, and is usable also as a low calorie bulking agent in foods, so that it is usable in any food wherein dextrin and maltodextrin are usually usable. More specifically, the indigestible dextrin is effectively usable for liquid or powdery beverages such as coffee, black tea, cola and juice; baked products such as bread, cookies, crackers, cakes, pizza and pies; noodles such as wheat noodles, Chinese noodles and buckwheat noodles; pasta such as spaghetti, macaroni and fettuccine; confectionery such as candies, chocolate and chewing gum; doughnut, potato chips and like fried cakes or foods; ices such as ice cream, shakes and sherbets; daily products such as cream, cheese, milk powder, condensed milk, creamy powder, coffee whitener and milk beverages; chilled desserts such as custard pudding, yoghurt, drinkable yoghurt, jelly, mousse and Bavarian; retorted pouched or canned foods such as soups, stew, gratin and curries; seasonings such as bean paste, soy sauce, Worceter sauce, ketchup, mayonnaise, dressing, bouillon and roux; processed meat products such as ham, sausage, hamburger, meatball and corned beef, and these products as frozen; frozen processed foods such as pilafs, croquettes, omelets and doria; processed fishery products such as artificial boiled crab paste and boiled fish paste; processed agricultural products such as dried mashed potatoes, jam, marmalade, peanut butter and peanut; others including food boiled down in soy, rice cakes, rice snacks and fast foods; alcoholic beverages such as wines, cocktails, fizzes and liqueur.

However, the dextrin is difficult to use in emulsified foods of the W/O type, such as margarin, since the dextrin incorporated therein is liable to separate off during preservation.

Further when serving as a low calorie bulking agent, the dextrin can be added to the food in an amount which is not limited insofar as the quality of the food is not impaired. However, if adults in good health take the low calorie bulking agent in an amount of 2$g$/$kg$ body weight by way of foods of the invention, diarrhea will occur in half of them, so that the amount of the agent to be taken is preferably not greater than half of this value, i.e., up to about 1$g$/$kg$ body weight.

Nevertheless, since the influence on physiological activities differs from person to person, it is most desirable to alter the amount in view of the effect achieved by the ingestion of the low calorie foods.

To check the indigestible dextrin for characterisitics when it is used in foods, chiefly the product of Example 6 was used in the foolowing experiments to obtain characterisitic dats.

Experimental Example 12

A sensory test was conducted to determine the sweetness of the indigestible dextrin in comparison with that of other saccharides and maltodextrin of DE25, with the sweetness of sucrose taken as 100. Table 19 shows the result.

Table 19

| Sample | Sweetness |
|---|---|
| Sugar | 100 |
| Glucose | 65 |
| Sorbitol | 50 |
| Maltodextrin (DE25) | 25 |
| Example 1 | 10 |
| Example 3 | 15 |
| Example 4 | 23 |

The indigestible dextrin is about 10 in sweetness, tasting slightly sweet.

Experimental Example 13

A 30% solution of indigestible dextrin was checked for viscosity at temperatures of 10 to 80°C using a Brookfield type viscosimeter. FIG.1 shows the result along with the corresponding values of sucrose, gum arabic and maltodextrin.

The symbols in FIG.1 stand for the following.

● :   indigestible dextrin of Example 6
▲ :   sucrose
■ :   maltodextrin
○ :   gum arabic

The indigestible dextrin is comparable to maltodextrin in voscosity. This indicates that the indigestible dextrin is usable in foods without entailing a great increase in viscosity.

### Experimental Example 14

Coloration due to heating in the presence of amino acid

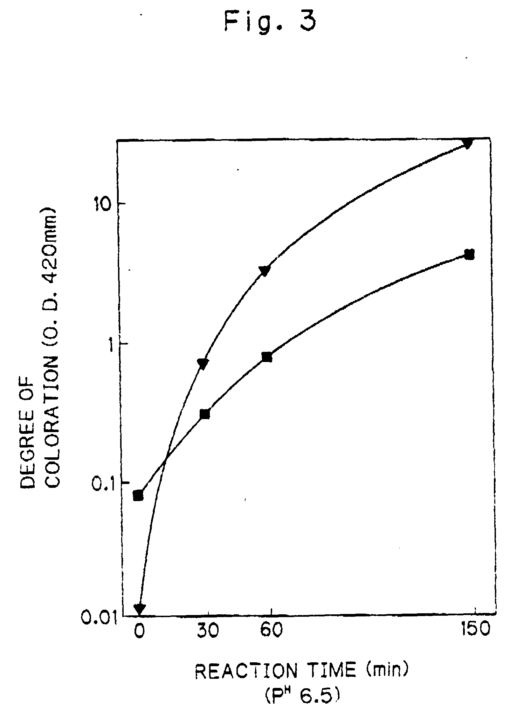

To 10% aqueous solution of indigestible dextrin was added 1% (based on solids) on glycine, and the mixture was heated at 100°C for 150 minutes and checked for changes in the degree of coloration. The results achieved at pH of 4.5 and pH of 6.5 are shown in FIGS.2 and 3, respectively. The same symbols as in FIG.1 were used in these drawings.

The indigestible dextrin is not greatly different in the increase of coloration degree from glucose or maltose. This indicates that the indigestible dextrin is usable generally in the same manner as these materials.

### Experimental Example 15

Freezing and thawing

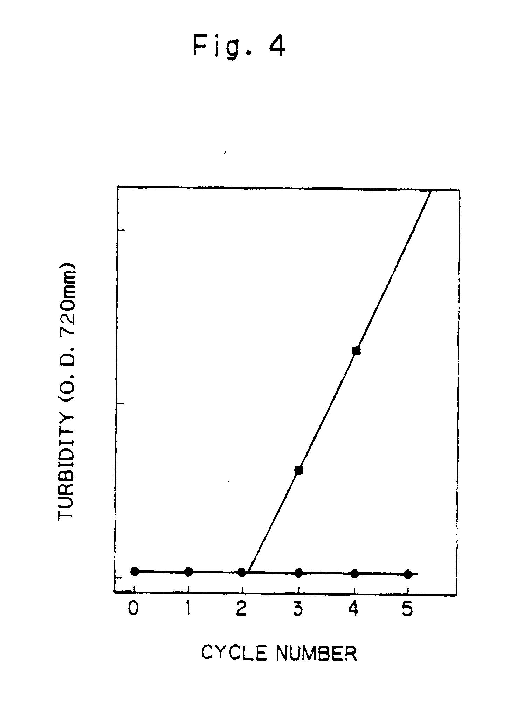

A 30% aqueous solution of indigestible dextrin was subjected to five repeated freeze-thaw cycles and then checked for resulting turbidity. FIG.4 shows the result along with the result achieved by maltodextrin. The symbols used in FIG.4 have the same meaning as in FIG.1.

The indigestible dextrin is much less than maltodextrin in increases in turbidity and is therefore very suitable for use in frozen foods.

### Experimental Example 16

Freezing point depression

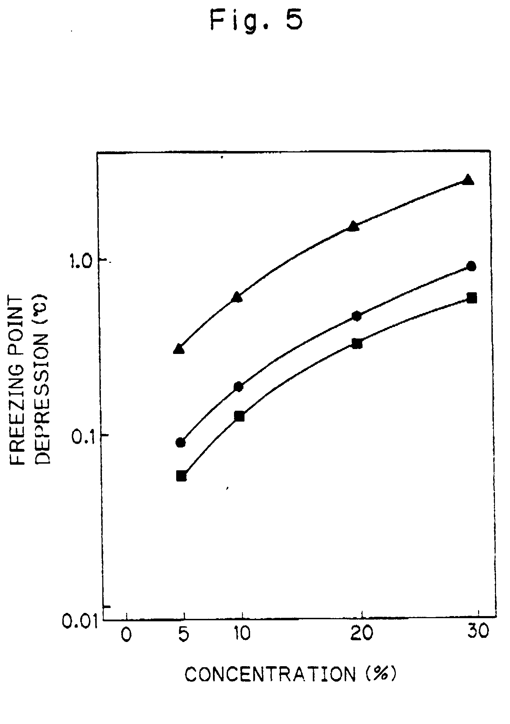

FIG.5 shows the result obtained by checking 5 to 30% aqueous solutions of indigestible dextrin for freezing point depression along with the result achieved by sucrose and maltodextrin. The symbols in FIG.5 are the same as in FIG.1.

The indigestible dextrin is generally intermediate between sugar and maltodextrin in the degree of freezing point depression and is therefore suited to use in ices and the like.

### Experimental Example 17

Hygroscopicity

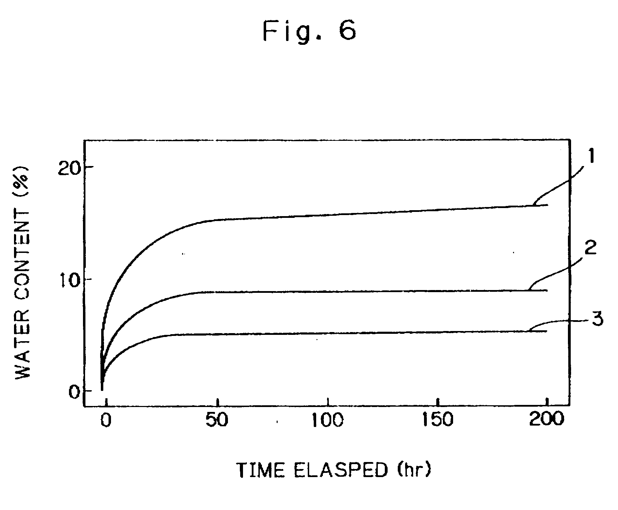

The indigestible dextrin was made anhydrous by drying and then allowed to stand in a constant-humidity container at 20°C and a relative humidity of 81%, 52% or 32% for 200 hours. FIG. 6 shows the hygroscopicity of the indigestible dextrin thus determined.

In FIG.6, the results obtained at R.H. 81%, R.H. 52% and R.H. 32% are indicated at (1), (2) and (3) , respectively.

The water content of the indigestible dextrin will not exceed 18% even if preserved for a long period of time. The indigestible dextrin is therefore suited to use in powdery foods.

### Example 5

Food example 1

A blackberry liquor was prepared according to the recipe of Table 69 by immersing blackberries in distilled spirits for 40 days, discarding the blackberries and then aging the spirits for 2 months.

Table 20

| Black berry liquor | Control | Example |
|---|---|---|
| Black berry | 57.0 | 57.0 |
| Sugar | 34.2 | 15.0 |
| Dextrin (Example 3) | - | 19.1 |
| Aspartame | - | 0.1 |

Table 20   (continued)

| Black berry liquor | Control | Example |
|---|---|---|
| White liquor (70 proof) | 65.8 | 65.8 |
| Dietary fiber | 0 | 6.05 |
| Dietary fiber/100$g$ | 0 | 6.05 |

**Claims**

1. An alcoholic beverage, which contains an indigestible dextrin prepared by adding 3-10 wt % of a 1% hydrochloric acid solution to corn starch ; stirring and aging the mixture in a mixer, heating the mixture at 150-200°C ; dissolving the resulting pyrodextrin in water to a concentration of 20-45% ; adjusting the pH of the solution to 5.5-6.5 ; hydrolysing the solution with $\alpha$-amylase at 80-90°C ; adjusting the pH of the solution to 4-6 ; hydrolyzing the solution with glucoamylase at 55-60°C ; lowering the pH of the resulting hydrolysate to about 3.5, heating it to about 80°C and purifying it ; concentrating the hydrolysate to a concentration of about 50 % and subjecting it to ion-exchange chromatography to remove at least half of the glucose formed therefrom, and which comprises a fraction other than glucose, wherein

   (A) said fraction contains at least 90 % of an indigestible component,
   (B) said fraction containing 25 to 35% of glucose residues having $1 \rightarrow 4$ glucosidic linkage only,
   (C) said fraction has a number average molecular weight of 900 to 1300,
   (D) said fraction has a number average molecular weight Y calculated from the equation : $Y = 3067 - 71.309 \cdot X$ wherein X is the amount (in % based on said fraction) of glucose residues having $1 \rightarrow 4$ glycosidic linkage only as quantitatively determined by "Hakomori's methylation method," said calculated value Y being in the range of variations of up to 20 % from the number average molecular weight as actually measured,
   (E) the ratio of the weight average molecular weight of said fraction to the number average molecular weight thereof being at least 25:1, and
   (F) the content of indigestible component and X are calculated as defined in the specification.

2. An alcoholic beverage as defined in claim 1 wherein the fraction other than glucose of the indigestible dextrin contains 25 to 30 % of glucose residues having $1 \rightarrow 4$ glycosidic linkage only and has a number average molecular weight of 990 to 1300.

3. An alcoholic beverage as defined in claim 1 or 2 wherein the indigestible dextrin contains up to 33 % of glucose and at least 75 % of an indigestible component.

4. An alcoholic beverage as defined in any one of claims 1 to 3 wherein the fraction other than glucose of the indigestible dextrin contains at least 20 % of dietary fiber and wherein the fraction containing glucose contains at least 13 % of dietary fiber.

5. An alcoholic beverage as defined in claim 4 wherein said fraction other than glucose is 1.6 to 1.8 kcal/g in caloric value 1 and the fraction containing glucose is 1.6 to 2.6 kcal/g in caloric value 1, wherein the said caloric value 1 is calculated as defined in the specification.

6. An alcoholic beverage as defined in claim 4 wherein said fraction other than glucose is 1 to 1.2 kcal/g in caloric value 2 and the fraction containing glucose is 1 to 2 kcal/g in caloric value 2, wherein the said caloric value 2 is calculated as defined in the specification.

7. An alcoholic beverage as defined in one of claims 1 to 6, which is a wine, a cocktail, a fizz or a liquor.

**Patentansprüche**

1. Alkoholisches Getränk, das ein unverdauliches Dextrin enthält, hergestellt durch die Zugabe von 3 bis 10 Gew.-% einer 1%igen Chlorwasserstoffsäurelösung zu Maisstärke, Rühren und Reifenlassen des Gemisches in einem Mischer, Erhitzen des Gemisches bei 150 bis 200°C; Auflösen des entstandenen Pyrodextrins in Wasser bis zu einer Konzentration von 20 bis 45 %, Einstellen des pH-Werts der Lösung auf 5,5 bis 6,5; Hydrolyse der Lösung mit α-Amylase bei 80 bis 90°C, Einstellen des pH-Werts der Lösung auf 4 bis 6; Hydrolyse der Lösung mit Glucoamylase bei 55 bis 60°C; Senken des pH-Werts des entstandenen Hydrolysats auf ungefähr 3,5, Erhitzen auf ungefähr 80°C und Reinigung des Hydrolysats; Konzentrieren des Hydrolysats auf eine Konzentration von ungefähr 50 % und Durchführen von einer Ionenchromatographie mit dem Hydrolysat, um mindestens die Hälfte der gebildeten Glucose daraus zu entfernen, und eine von Glucose verschiedene Fraktion umfaßt, wobei

(A) die Fraktion mindestens 90 % einer unverdaulichen Komponente enthält,
(B) die Fraktion mindestens 25 bis 35 % Glucoserückstände enthält, die ausschließlich 1 → 4 Glycosidbindungen aufweist.
(C) die Fraktion ein Zahlenmittel des Molekulargewichts von 900 bis 1300 aufweist,
(D) die Fraktion ein Zahlenmittel des Molekulargewichts aufweist, das durch die Gleichung: Y = 3067-71,309 · X berechnet werden kann,
wobei X die Menge (in %, bezogen auf die Fraktion) der Glucoserückstände ist, die ausschließlich 1 → 4 Glycosidbindungen aufweist, wie sie durch das "Methylierungsverfahren von Hakomori" quantitativ bestimmt werden kann, wobei der berechnete Wert Y in einem Abweichungsintervall von bis zu 20 % um das tatsächlich gemessene Zahlenmittel des Molekulargewichts liegt,
(E) das Verhältnis des Massenmittels des Molekulargewichts der Fraktion im Verhältnis zu ihrem Zahlenmittel des Molekulargewichts mindestens 25:1 ist, und
(F) der Gehalt des unverdaulichen Bestandteiles sowie X wie in der Beschreibung definiert bestimmt werden.

**2.** Alkoholisches Getränk wie in Anspruch 1 definiert, wobei die von Glucose verschiedene Fraktion des unverdaulichen Dextrins 25 bis 30 % Glucoserückstände enthält, die ausschließlich 1 → 4 Glycosidbindungen und ein Zahlenmittel des Molekulargewichts von 990 bis 1300 aufweist.

**3.** Alkoholisches Getränk wie in Ansprüchen 1 oder 2 definiert. wobei das unverdauliche Dextrin bis zu 33 % Glucose und mindestens 75 % eines unverdaulichen Bestandteils enthält.

**4.** Alkoholisches Getränk wie in einem der Ansprüche 1 bis 3 definiert, wobei die von Glucose verschiedene Fraktion des unverdaulichen Dextrins mindestens 20 % einer Diätfaser enthält, und wobei die Glucose enthaltende Franktion mindestens 13 % einer Diätfaser enthält.

**5.** Alkoholisches Getränk wie in Anspruch 4 definiert, wobei die von Glucose verschiedene Fraktion einen Kalorienwert 1 von 1,6 bis 1,8 kcal/g aufweist und die Glucose enthaltende Fraktion einen Kalorienwert 1 von 1,6 bis 2,6 kcal/g aufweist, wobei der Kalorienwert 1 wie in der Beschreibung definiert bestimmt wird.

**6.** Alkoholisches Getränk wie in Anspruch 4 definiert, wobei die von Glucose verschiedene Fraktion einen Kalorienwert 2 von 1 bis 1,2 kcal/g aufweist, und die Glucose enthaltende Fraktion einen Kalorienwert 2 von 1 bis 2 kcal/g aufweist, wobei der Kalorienwert 2 wie in der Beschreibung definiert bestimmt wird.

**7.** Alkoholisches Getränk wie in einem der Ansprüche 1 bis 6 definiert. wobei das Getränk ein Wein, ein Cocktail. ein spritziges Getränk oder eine Spirituose ist.

**Revendications**

**1.** Boisson alcoolique, contenant une dextrine indigestible qui est préparée par un procédé selon lequel on ajoute 3 à 10 % en poids d'une solution d'acide chlorhydrique à 1 % à de l'amidon de maïs ; on agite et on fait vieillir le mélange dans un mélangeur, on chauffe le mélange à 150-200°C ; on dissout la pyrodextrine obtenue dans de l'eau à une concentration de 20-45 % ; on amène le pH de la solution à 5,5-6,5 ; on hydrolyse la solution avec une α-amylase à 80-90°C ; on amène le pH de la solution à 4,6 ; on hydrolyse la solution avec une glucoamylase à 55-60°C ; on abaisse le pH de l'hydrolysat obtenu à environ 3,5, on le chauffe à environ 80°C et on le purifie ; on concentre l'hydrolysat à une concentration d'environ 50 % et on le soumet à une chromatographie par échange d'ions pour en éliminer au moins la moitié du glucose formé, et qui comprend une fraction différente du glucose. dans laquelle

(A) ladite fraction contient au moins 90% d'un constituant indigestible,
(B) ladite fraction contient 25 à 35 % de résidus de glucose n'ayant que des liaisons glycosidiques 1 → 4,

(C) ladite fraction a un poids moléculaire moyen en nombre de 900 à 1 300,

(D) ladite fraction a un poids moléculaire moyen en nombre Y calculé par l'équation Y = 3067 - 71,309 X où X est la quantité (en % par rapport à ladite fraction) de résidus de glucose ne contenant que des liaisons glycosidiques 1 → 4, déterminée quantitativement par la "méthode de méthylation de Hakomori", ladite valeur calculée Y pouvant présenter une variation d'au plus 20 % par rapport au poids moléculaire moyen en nombre réellement mesuré ;

(E) le rapport du poids moléculaire moyen en poids de ladite fraction à son poids moléculaire moyen en nombre est d'au moins 25:1, et

(F) la teneur en constituant indigestible et X sont calculés de la manière définie dans la description.

2. Boisson alcoolique selon la revendication 1, dans laquelle la fraction différente du glucose de la dextrine indigestible contient 25 à 30 % de résidus de glucose n'ayant que des liaisons glycosidiques 1 → 4 et a un poids moléculaire moyen en nombre de 990 à 1 300.

3. Boisson alcoolique selon la revendication 1 ou 2, dans laquelle la dextrine indigestible contient au plus 33 % de glucose et au moins 75 % d'un constituant indigestible.

4. Boisson alcoolique selon l'une quelconque des revendications 1 à 3, dans laquelle la fraction différente du glucose de la dextrine indigestible contient au moins 20 % de fibres alimentaires, et la fraction contenant du glucose contient au moins 13 % de fibres alimentaires.

5. Boisson alcoolique selon la revendication 4, dans laquelle ladite fraction différente de glucose a une valeur calorique 1 de 1,6 à 1,8 kcal/g et la fraction contenant du glucose a une valeur calorique 1 de 1,6 à 2,6 kcal/g, ladite valeur calorique 1 étant calculée comme défini dans la description.

6. Boisson alcoolique selon la revendication 4, dans laquelle ladite fraction différente du glucose a une valeur calorique 2 de 1 à 1,2 kcal/g et la fraction contenant du glucose a une valeur calorique 2 de 1 à 2 kcal/g, ladite valeur calorique 2 étant calculée comme défini dans la description.

7. Boisson alcoolique selon l'une quelconque des revendications 1 à 6, qui est un vin, un cocktail, une boisson gazeuse ou une liqueur.

# Fig. 1

# Fig. 2

Fig. 3

# Fig. 4

# Fig. 5

Fig. 6